# EUROPÄISCHE PATENTANMELDUNG

(11) **EP 2 229 932 A1**
(43) Veröffentlichungstag der Anmeldung: **22.09.2010**
(21) Anmeldenummer: 09003954.6
(22) Anmeldetag: 19.03.2009
(51) Int. Cl.: A61K 8/06, A61K 8/37, A61K 8/60, A61Q 15/00

(54) **Antitranspiration in Form einer Mikroemulstion und Doppelemulsion**

(71) Anmelder: Cognis IP Management GmbH, 40589 Düsseldorf (DE)
(72) Erfinder: Hloucha, Matthias, 50677 - Köln (DE); Gehm, Esther Ricarda, 67655 - Kaserslautern (DE); Menzer, Jasmin, 40789 - Monheim (DE); Weichold, Catherine, 52072 - Aachen (DE); Schulte, Petra, 50733 - Köln (DE)

(57) **Zusammenfassung**

Die vorliegende Erfindung betrifft stabile Antitranspirant/Desodorant-Mikroemulsionen und -Doppelemulsionen mit guten sensorischen Eigenschaften, die ohne ethoxylierte Substanzen formulierbar sind.

## Beschreibung

### Gebiet der Erfindung

Die vorliegende Erfindung betrifft stabile Antiperspirant/Desodorant-Mikroemulsionen mit guten sensorischen Eigenschaften, die ohne ethoxylierte Substanzen formulierbar sind sowie Doppelemulsionen, die diese Mikroemulsionen enthalten.

### Stand der Technik

AP/Deo-Formulierungen des Marktes sind in der Regel Ethylenoxid-haltig, da die in den Formulierungen verwendeten Emulgatoren üblicherweise Ethylenoxid (EO) enthalten, was aus ökologischer Sicht kritisch gesehen wird, auch wenn sie hinsichtlich der Schutzwirkung gegen Schweißgeruch eine ausreichende Leistung haben.

DE 19530220 A1 offenbart Antiperspirant/Desodorant-Mikroemulsionen, enthaltend eine Ölphase, nichtionische Emulgatoren und wahlweise Coemulgatoren mit sehr breiten Mengenbreichen für die Emulgatoren und Coemulgatoren. Als Emulgatoren werden vorzugsweise Alkypolyglycoside (auch: "Alkyloligoglycoside") eingesetzt.

Aufgabe der vorliegenden Erfindung war es, Antiperspirant/Desodorant-Formulierungen bereitzustellen, die ohne ethoxylierte Rohstoffe formulierbar sind, und die gegenüber bekannten Antiperspirant/Desodorant-Formulierungen eine verbesserte Stabilität zeigen, wobei die Formulierungen auch gute sensorische Eigenschaften aufweisen.

Kleine Teilchengrößen der dispergierten Phase tragen bei Emulsionen zu guten sensorischen Eigenschaften und guter Verträglichkeit bei. Eine weitere Aufgabe der vorliegenden Erfindung war es daher, Antiperspirant/Desodorant-Formulierungen bereitzustellen, deren dispergierte Phase möglichst feinteilig ist.

### Beschreibung der Erfindung

Überraschenderweise wurde gefunden, dass eine Mikroemulsion oder Doppelemulsion der vorliegenden Erfindung dieses Problem löst.

Gegenstand der vorliegenden Erfindung ist eine Antiperspirant/Desodorant-Mikroemulsion mit folgenden Bestandteilen:
(A) 10-20 Gew.-% Alkyl- und/oder Alkenyloligoglykosid(en),
(B) 5-10 Gew.-% Fettsäure-Polyol-Partialester, bevorzugt Glycerinmonooleat,
(C) 10-30 Gew.-% Ölphase, und
(D) 5-30 Gew.-% Anfiperspirant-/Desodorant-Wirkstoff, jeweils bezogen auf das Gesamtgewicht der Mikroemulsion,
wobei das Gewichtsverhältnis von (A) zu (B) mindestens 2.0 beträgt.

Gegenstand der vorliegenden Erfindung ist weiterhin eine Doppelemulsion, welche die erfindungsgemäße Mikroemulsion als dispergierte Phase und eine Ölphase als äußere Phase enthält.

Insbesondere betrifft die vorliegende Erfindung eine Doppelemulsion mit folgenden Bestandteilen:
(A) 6-15 Gew.-% Alkyl- und/oder Alkenyloligoglykosid(en),
(B) 3-6 Gew.-% Fettsäure-Polyol-Partialester, bevorzugt Glycerinmonooleat,
(C) 5-15 Gew.-% wasserunlösliche Phase,
(D) 5-20 Gew.-% Antiperspirant-/Desodorant-Wirkstoff,
(E) 20-40 Gew.-% Silikonöl, und
(F) 2-8 Gew.-% Silikonölemulgator, jeweils bezogen auf das Gesamtgewicht der Doppelemulsion,
wobei das Gewichtsverhältnis von (A) zu (B) mindestens 2.0 beträgt.

Mikroemulsionen sind optisch isotrope, thermodynamisch stabile Systeme, die spontan entstehen und die eine wasserunlösliche Komponente (Ölphase), Emulgatoren und Wasser enthalten. Die Tröpfchengröße von Mikroemulsionen liegt üblicherweise im Bereich von unter 100 nm, sodass sie üblicherweise transparent sind. Die erfindungsgemäße Teilchengröße der dispergierten Phase der Mikroemulsion beträgt erfindungsgemäß ebenfalls bevorzugt unter 100 nm, bestimmt mittels eines Coulter LS.

Doppelemulsionen sind Emulsionen, bei denen die dispergierte Phase selbst aus Emulsionströpfchen gebildet ist. Sie können hergestellt werden, indem eine Emulsion in eine mit der Emulsion nicht mischbare Phase getropft werden. Bei einer Doppelemulsion liegen damit Tröpfchen mit einer äußeren und einer inneren Phase vor, wobei diese Tröpfchen wiederum in einer äußersten Phase dispergiert sind. Erfindungsgemäß handelt es sich um O/W/O-Emulsionen. Die erfindungsgemäßen Doppelemulsionen können transparent oder trüb sein.

Die vorliegende Erfindung beinhaltet auch ein Verfahren zur Verbesserung der Stabilität von Mikroemulsionen.

### Komponente (A) - Emulgator

Als Emulgator (Komponente (A)) werden erfindungsgemäß Alkyl- und/oder Alkenyl(oligo)glykosid(e) mit 8 bis 22 Kohlenstoffatomen im Alk(en)ylrest eingesetzt.

C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Ein bevorzugtes Beispiel ist Laurylglucosid. Ebenfalls erfindungsgemäß bevorzugt ist Komponente (A) ein Alkyloligoglykosidgemisch mit einem Verhältnis von C12-Alkylrest zu C14-Alkylrest von etwa 7: etwa 3.

Die Mikroemulsionen enthalten die Komponente (A) in Mengen von 10 bis 20, vorzugsweise 12 bis 18, insbesondere 14 bis 18 Gew.% - bezogen auf das Gesamtgewicht der Mikroemulsion.

### Komponente (B) - Coemulgator

Komponente (B) wirkt als Coemulgator und ist erfindungsgemäß ein Fettsäure-Polyol-Partialester.

Unter Fettsäure-Polyol-Partialestem sind erfindungsgemäß die Produkte der Veresterung von Fettsäuren mit 8 bis 22 C-Atomen, vorzugsweise 12 bis 22 C-Atomen mit polyfunktionellen Alkoholen der Funktionalität 3 bis 10, vorzugsweise 3 bis 6 und insbesondere bevorzugt 3 oder 4, zu verstehen.

Besonders bevorzugt sind dabei solche Fettsäure-Polyol-Partialester, bei denen lediglich eine OH-Funktionalität mit einer geeigneten Fettsäure verestert ist. Die bei einer solchen Veresterung entstehenden Gemische sollten vorteilhafterweise 35 bis 96% Monoester, 1 bis 50% Diester und 0,1 bis 20% Tri- oder höhere Ester enthalten.

Wählt man Glycerin als Polyol, so lassen sich die Partialester auf besonders einfache Weise durch Umesterung natürlicher Fette oder Öle mit einem Überschuss an Glycerin erhalten. Geeignete natürliche Fette und Öle stellen beispielsweise Rindertalg, Schweinschmalz, Palmöl, Sonnenblumenöl oder Sojaöl, bevorzugt solche natürlichen Fette oder Öle mit einem besonders hohen Anteil an Ölsäure dar. Als Polyole eignen sich z. B. Propylenglycol, Glycerin, Erythrittrimethylolpropan, Pentaerythrit, Sorbit, Diglycerin, Methylglycosid oder auch Aldosen wie z. B. Glucose oder Mannose.

Geeignete Fettsäure-Polyol-Partialester sind z. B. die technischen Glycerin- oder Sorbitan-Monoester von Myristinsäure, Palmitinsäure, Stearinsäure und Ölsäure oder von technischen Kokosfettsäure C12-C18-Schnitten. Bevorzugt werden Sorbitanmonooleat, Stearinsäuremonoglycerid sowie insbesondere bevorzugt Öisäuremonoglycerid verwendet.

Erfindungsgemäß bevorzugt ist ein Glycerinoleat, besonders bevorzugt ein Glycerinmonooleat. Bevorzugt ist auch ein Glycerinoleatgemisch mit hohem Glycerinmonooleatanteil. Bevorzugt ist als Komponente (B) ein Glycerinoleat mit mehr als 80 Gew.-% ungesättigten C18-Fettsäuren enthalten, wobei der Monoesteranteil mehr als 90 Gew.-% beträgt.

Die Mikroemulsionen enthalten die Komponente (B) in Mengen von 5 bis 10 Gew.-%, vorzugsweise 6 bis 10, insbesondere 6 bis 10 Gew.% - bezogen auf das Gesamtgewicht der Mikroemulsion.

Erfindungsgemäß ist es notwendig, dass das Gewichtsverhältnis der Komponente (A) zur Komponente (B) 2.0 oder größer ist, bevorzugt 2,0 bis 4.0, bevorzugter 2.0 bis 3.0 und 2.0 bis 2.5.

### Wasserunlösliche Komponente bzw. Ölphase (Komponente (C))

Die Ölphase kann aus Ölen, Fetten, Wachsen sowie beliebige Mischungen daraus gebildet sein.

Als wasserunlösliche Ölkomponente (C) eignen sich alle bei 30°C flüssigen Fettstoffe oder Fettstoffgemische, d. h. auch Gemische aus flüssigen und darin gelösten, festen Fettstoffen oder Paraffinen, solange diese Gemische bei 30°C flüssig sind oder deren Viskosität (20°C) unter 20 Pas liegt (gemessen mit einem Brookfield Rotationsviskosimeter Typ RVF, Spindel 4, 10 UpM), wie z. B. flüssige Kohlenwasserstoffe, Dialkylether, Fettsäureester mit 12 - 44 C-Atomen, Dialkylcarbonate, Guerbetalkohole und Siliconöle oder deren Mischungen.

Unter dem Begriff "Öle" (synonym verwendet: Ölkomponente) werden wasserunlösliche, bei 30°C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz.

Als Ölkomponenten sind beispielsweise die nachstehend genannten Verbindungsklassen geeignet, soweit diese bei 30°C flüssig sind. So z.B. Guerbetalkohole auf Basis von Fettalkoholen mit 6 bis 18, vorzugsweise 8 bis 10 Kohlenstoffatomen (z. B. Eutanol^{®} G) , Ester von linearen C₆-C₂₂-Fettsäuren mit linearen oder verzweigten C₆-C₂₂-Fettalkoholen (z.B. Cetiol^{®} Sensoft) bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat und Hexyldecylstearat (Eutanol® G 16 S). Daneben eignen sich Ester von linearen C₆-C₂₂-Fettsäuren mit verzweigten Alkoholen, insbesondere 2- Ethylhexanol, Ester von C₃-C₃₈-Alkylhydroxycarbonsäuren mit linearen oder verzweigten C₆-C₂₂-Fettalkoholen - insbesondere Dioctylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1 ,3-Dialkylcyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Hydagen® HSP, Sovermol® 750, Sovermol® 1102) und/oder aliphatische bzw. naphthenische Kohlenwasserstoffe, wie z.B. wie Mineralöl, Vaseline, Petrolatum, Squalan, Squalen oder Dialkylcyclohexane.

Als Ölkomponenten eignen sich auch Polycarbonate, wie beispielsweise in WO 03/041676 beschrieben, auf die hier ausdrücklich Bezug genommen wird.

Als Polycarbonate besonders geeignet ist das unter der INCI Bezeichnung Hydrogenated Dimer Dilinoleyl / Dimethylcarbonate Copolymer, welches als Handelsprodukt Cosmedia^{®} DC von Cognis GmbH erhältlich ist.

Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen.

Erfindungsgemäß einsetzbar sind u. a. auch Kohlenwasserstoffe, vorzugsweise mit einer Kettenlänge 8 bis 40 C-Atomen. Sie können verzweigt oder unverzweigt sein, gesättigt oder ungesättigt. Unter diesen sind verzweigte, gesättigte C₈-C₄₀-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8-Diethyldodecan und/oder mehr als 1 Gew.-% Didecen enthalten.

Erfindungsgemäß als Ölphase besonders bevorzugt sind Di-n-alkylether, wobei Di-n-octylether bzw. Dicaprylylether besonders bevorzugt ist. Die Di-n-alkylether sind dabei in einem Anteil von mindestens 30 Gew.-%, bevorzugt mindestens 40 Gew.-% und besonders bevorzugt mindestens 50 Gew.-% , bezogen auf die Ölphase, enthalten. Besonders vorteilhafte Ausführungsformen der Mikroemulsionen enthalten die Di-n-alkylether in der Ölphase in einem Anteil von wenigstens 60 Gew.-%, wobei es vorteilhaft sein kann, den Anteil an Ether auf beispielsweise 80 bis 90 Gew.-%, bezogen auf die Ölphase, zu erhöhen. Gegebenenfalls kann der Anteil der Ether an der Ölphase sogar 100 Gew.-% betragen, wobei die Phasenbreite der Mikroemulsion besonders groß ist.

Es kann erfindungsgemäß eine Öl oder ein Gemisch aus mehreren Ölkomponenten verwendet werden.

Unter dem Begriff Wachs (synonym verwendet: Wachskomponente) werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a.
Fette (Triglyceride), sowie natürliche und synthetische Wachse oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar sind so genannte gehärtete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina® HR im Handel ist. Ebenso geeignet sind Glycerintristearat, Glycerintribehenat (z. B. Syncrowax® HRC), Glycerintripalmitat oder die unter der Bezeichnung Syncrowax® HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis GmbH vermarkteten Produkte Novata AB und Novata B (Gemisch aus C₁₂-C₁₈-Mono-, Di- und Triglyceriden) sowie Cutina MD oder Cutina GMS (Glycerylstearat).

Weitere Wachskomponenten sind aus der Gruppe der C6-C22-Fettsäureester des Pentaerythrits, des Dipentaerythrits, des Tripentaerythrits oder eines beliebigen Gemisches dieser Ester, die einen Schmelzpunkt von wenigstens 30°C aufweisen. Ein bevorzugtes Pentaerythritsestergemisch besteht aus einem Anteil von 5 - 35 Gew.-% Monoester, 20 - 50 Gew.-% Diester und 25 - 50 Gew.-% Triester, und ggf. Tetraester. Besonders bevorzugt ist ein Gehalt an 10 - 25 Gew.-% Monoester, 25 - 40 Gew.-% Diester und 30 - 45 Gew.-% Triester, und ggf. Tetraester und ganz besonders bevorzugt 12 - 19 Gew.-% Monoester, 25 - 35 Gew.-% Diester und 30 - 40 Gew.-% Triester und 6 - 11 Gew.-% Tetraester. Zu dem erfindungsgemäß einsetzbaren Pentaerythritsestergemisch zählt das von der Cognis GmbH vermarktete Produkt Cutina^{®} PES.

Zu den erfindungsgemäß als Wachskomponente einsetzbaren Fettalkoholen zählen die C₁₂-C₅₀-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C₁₄-C₂₂-Fettalkohole, die beispielsweise von der Cognis GmbH unter der Bezeichnung Lanette 18 (C₁₈-Alkohol), Lanette 16 (C₁₆-Alkohol), Lanette 14 (C₁₄-Alkohol), Lanette O (C₁₆/C₁₈-Alkohol) und Lanette 22 (C₁₈/C₂₂-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C₁₄-C₄₀-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche **pflanzliche Wachse,** wie Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse,** wie z. B. Ceresin und Ozokerit oder die petrochemischen Wachse, wie z. B. Petrolatum, Paraffinwachse und Mikrowachse. Als Wachskomponente sind auch **chemisch modifizierte Wachse,** insbesondere die Hartwachse, wie z. B. Montanesterwachse, Sasolwachse und hydrierte Jojobawachse einsetzbar. Zu den **synthetischen Wachsen,** die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder - stearat kommen hierfür in Frage.

Als Wachse eignen sich weiterhin **Perlglanzwachse.** Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Es kann erfindungsgemäß ein Wachs oder ein Gemisch aus mehreren Wachsen verwendet werden.

Die Ölphase macht erfindungsgemäß einen Anteil von 10 bis 30 Gew.-% der Mikroemulsion aus.

In einer bevorzugten Ausführungsform der vorliegenden Erfindung ist das Gewichtsverhältnis der Komponente (C) zu (B) 2.0 oder größer, bevorzugt etwa 2.0 bis 2.5.

### Antiperspirant/Desodorant-Wirkstoff - Komponente (D)

Erfindungsgemäß sind als Antiperspirant/Desodorant-Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

Der Antiperspirant/Desodorant-Wirkstoff oder die Antiperspirant/Desodorant-Wirkstoffe ist/sind in erfindungsgemäß in einem Anteil von 5 bis 30 Gew.-%, bezogen auf das Gesamtgewicht der Mikroemulsion, bevorzugter 10 bis 30 Gew.-% und noch bevorzugter 15 bis 25 Gew.-%. Es kann ein einziger Antiperspirant/Desodorant-Wirkstoff oder es können mehrere Antiperspirant/Desodorant-Wirkstoffe enthalten sein. Im letzteren Fall beziehen sich die obigen Prozentangaben auf die Gesamtmenge der enthaltenen Antiperspirant/Desodorant-Wirkstoffe.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorhydrat und deren Komplexverbindungen eingesetzt, wie z.B. Locron L oder ACH-303 50% Solution, Rezal 67 oder AZ-7373 Powder, Rezal 36 GC oder AZG-7226 50% Solution.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäure-diethylester, Hydroxycarbonsäuren und deren Ester wie beispielsweise Citronensäure, Äpfelsäure, Weinsäure oder Weinsäurediethylester sowie Zinkglycinat.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen.

### Wässriger Anteil und weitere Bestandteile

Erfindungsgemäß wird der Restbestandteil der Mikroemulsion durch Wasser gebildet, und es können weitere übliche Bestandteile enthalten sein.

Typischerweise beträgt der Wassergehalt von 10 bis 50 Gew.-%, bezogen auf die Mikroemulsion, bevorzugt 10 bis 40 Gew.-%.

### Weitere Bestandteile

Neben den genannten Bestandteilen können andere, in Antitranspirantien/Desodorantien übliche Bestandteile in üblichen Anteilen enthalten sein. Hierzu gehören z. B. Tenside, Konservierungsmittel, biogene Wirkstoffe, Verdickungsmittel, Überfettungsmittel, Stabilisatoren, Polymere, Antioxidantien, Filmbildner, Quellmittel, Insektenrepellentien, Hydrotrope, Solubilisatoren, Parfümöle, Farbstoffe, Pigmente, UV-Filter etc.

Sollten weitere Bestandteile wasserunlöslich sein, sind sie der lipophilen Phase zuzurechnen.

Die erfindungsgemäßen Mikroemulsionen enthalten die weiteren Bestandteile üblicherweise in Mengen von insgesamt < 25 Gew.%, insbesondere < 20 Gew.%, bezogen auf das Gesamtgewicht der Mikroemulsion.

### Tenside

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekularteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächenaktive Substanzen verstanden, die einen HLB-Wert von größer 20 aufweisen.

Als oberflächenaktive Stoffe können anionische, nichtionische, kationische und/oder amphotere bzw. zwitterionische Tenside enthalten sein. In tensidhaltigen kosmetischen Zubereitungen ist vorzugsweise wenigstens ein anionisches Tensid enthalten.

Die erfindungsgemäßen Zubereitungen können die Tenside üblicherweise in einer Menge von 0 bis 40 Gew.-%., vorzugsweise 0,05 bis 30 Gew.-% insbesondere 0,05 bls 20 Gew.-%, vorzugsweise 0,1 bis 15 Gew.-% und insbesondere 0,1 bis 10 Gew.-%, bezogen auf das Gesamtgewicht der Zubereitung, enthalten.

Typische Beispiele für **nichtionische Tenside** sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind **ampholytische Tenside.** Unter ampholytischen Tensiden werden solche oberflächenaktiven Verbindungen verstanden, die außer einer C₈-C₁₈-Alkyl-oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest.

Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosirtate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantek^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Als **Konservierungsmittel** eignen sich beispielsweise Phenoxyethanol, Ethylhexylglycerin, Dicaprylyl Glycol, Formaldehydlösung, Parabene, Pentandiol, Mischungen aus Phenoxyethanol und Ethylhexylglycerin (wie sie beispielsweise unter dem Handelsnamen Euxyl PE 9010 erhältlich sind), oder Sorbinsäure sowie die unter der Bezeichnung Surfacine® bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung, Parabenen, organische Säuren und Mischungen daraus, gegebenenfalls in Kombination mit Pentandiol und/oder Ethylhexylglycerin bzw. Octandiol und Ethylhexylglycerin (Handelsname Sensiva SC 10).

In einer bevorzugten Ausführungsform der Erfindung enthalten die erfindungsgemäßen Mikroemulsionen als biogenen Wirkstoff mindestens eine Verbindung ausgewählt aus Vitaminen, Allantoin, Bisabolol und Pflanzenextrakten.

In einer bevorzugten Ausführungsform der Erfindung enthalten die erfindungsgemäßen Mikroemulsionen als biogenen Wirkstoff mindestens eine Verbindung ausgewählt aus Tocopherol, Tocopherolacetat, Tocopherolpalmitat, Ascorbinsäure, (Desoxy)Ribonucleinsäuren und deren Fragmentierungsprodukten, β-Glucanen, Retinol, Bisabolol, Allantoin, Phytantriol, Panthenol, AHA-Säuren, Aminosäuren, Ceramiden, Pseudoceramiden, essentiellen Ölen, Pflanzenextrakten, wie z. B. Aloe vera, Prunusextrakt, Bambaranussextrakt und Vitaminkomplexe und Mischungen daraus.

In einer Ausführungsform der Erfindung enthalten die erfindungsgemäßen Mikroemulsionen als weiteren Bestandteil mindestens ein Verdickungsmittel.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Als Insekten-Repellentien kommen beispielsweise N,N-Diethyl-m-toluamid, 1 ,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Es ist ferner möglich, dass die erfindungsgemäße Mikroemulsion einen oder mehrere Farbstoffe enthält.

Die Farbstoffe können sowohl synthetischen als auch natürlichen Ursprungs sein.
Eine Liste von geeigneten Farbstoffen findet sich in EP 1 371 359 A2, S.8, Z. 25-57, S.9 und S.10 sowie S.11, Z. 1 bis 54, auf welche hiermit explizit Bezug genommen wird.

Als Farbstoffe und Pigmente eignen sich insbesondere die gemäß Annex IV der Kommissions Direktive zugelassenen Farbstoffe und Pigmente (in der Fassung: Commission Directive 2007/22/EC of 17 April 2007 amending Council Directive 76/768/EEC, conceming cosmetic products, for the purposes of adapting Annexes IV and VI thereto to technical progress), auf die hiermit explizit Bezug genommen wird.

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- boman-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-[2(und 4)- (2-oxoborn-3-ylidenmethyl)benzyl]acrylamid Mexoryl SW)
➢ 2-(2H-Benzotriazol-2-yl)-4-methyl-6-(2-methyl-3-(1,3,3,3-tetramethyl-1-(trimethylsilyloxy)disiloxanyl)propyl) phenol (Mexoryl XL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)diimino] bis(benzoesäure-2- ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV-Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung **Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (SunJun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von **P. Finkel in SÖFW-Journal 122, 8/1996, S. 543-548 sowie Parf. Kosm. 80. Jahrgang, Nr. 3/1999, S.10 bis 16** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als Stabilisatoren können Metallsalze von Fettsäuren, wie z. B. Magnesium-, Aluminium- und/oder Zinkstearat bzw. -ricinoleat eingesetzt werden.

Die weiteren Bestandteile der Zubereitungen (wie z.B. Konservierungsmittel, kosmetische Wirkstoffe wie z.B. DHA - Dihydroxyaceton -, UV-Filter etc.) werden je nach ihrer Löslichkeit entweder über die Wasserphase oder über die lipophile Phase zugegeben.

### Doppelemulsion

Die vorliegende Erfindung beinhaltet auch eine Doppelemulsion, welche die oben beschriebene erfindungsgemäße Mikroemulsion als dispergierte Phase und eine Ölphase als äußere Phase enthält. Es handelt sich erfindungsgemäß um eine O/W/O-Emulsion. Die äußerste Phase wird dabei bevorzugt durch Silikonöl gebildet. Weiterhin ist bevorzugt ein Silikonölemulgator enthalten.

### Komponente (E) - Silikonöle

Als äußerste Phase werden bevorzugt Silikonöle verwendet. Sie können als cyclische und/oder lineare Silikonöle vorliegen. Silikonöle sind hochmolekulare synthetische polymere Verbindungen, in denen Silicium- Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 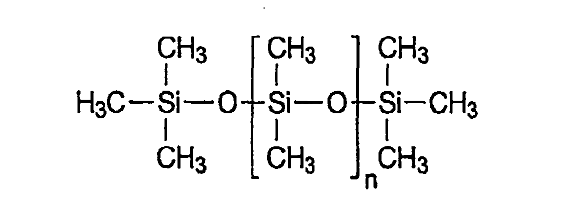 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handels- bezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo-trisiloxan, Polydimethylsiloxan, Poly (methylphenylsiloxan). Erfindungsgemäß besonders bevorzugte Silikone sind Dimethicon und Cyclomethicon.

Ein besonders bevorzugtes Silikonöl ist Cyclopentasiloxan, welches von Dow Coming unter der Bezeichnung DC 245 erhältlich ist.

### Komponente (F) - Silikonölemulgatoren

Die Silikonölemulgatoren können beispielsweise aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 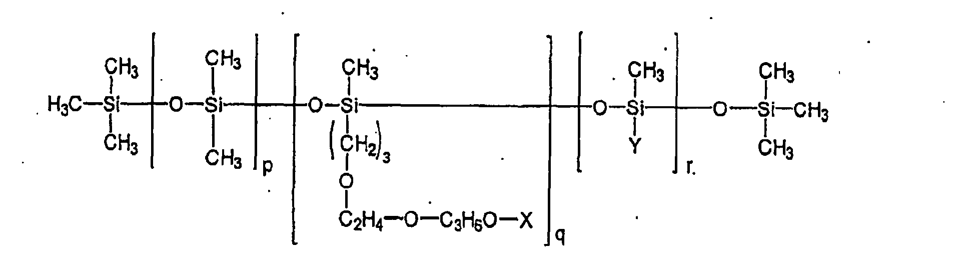 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für vorteilhaft im Sinne der vorliegenden Erfindung zu verwendende Silikonölemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein Beispiel für besonders vorteilhaft im Sinne der vorliegenden Erfindung zu verwendende Silikonölemulgatoren ist das Cetyl PEG/PPG-10/1 Dimethicone (Cetyl Dimethiconcopolyol), welches von Evonik Goldschmidt unter der Warenbezeichnung ABIL® EM 90 verkauft wird.

Ein weiteres Beispiel für vorteilhaft im Sinne der vorliegenden Erfindung zu verwendende Silikonölemulgatoren ist das Cyclomethicon Dimethiconcopolyol, welches von Evonik Goldschmidt unter der Warenbezeichnung ABIL®EM 97 und ABIL®WE 09 verkauft wird.

Weiterhin verwendbar ist z. B. der Emulgator Lauryl PEG/PPG-18/18 Methicone (Laurylmethiconcopolyol) herausgestellt, welcher unter der Warenbezeichnung Dow Coming® 5200 Formulation Aid von der Gesellschaft Dow Coming Ltd. erhältlich ist.

Ein weiterer vorteilhafter Silikonölemulgator ist, Octyl Dimethicon Ethoxy Glucosid der Firma Wacker.

Es können erfindungsgemäß auch Gemische dieser Silikonölemulgatoren verwendet werden.

Insbesondere betrifft die vorliegende Erfindung eine Doppelemulsion mit folgenden Bestandteilen:
(A) 6-15 Gew.-% Alkyl- und/oder Alkenyloligoglykosid(en),
(B) 3-6 Gew.-% Fettsäure-Polyol-Partialester, bevorzugt Glycerin(mono)oleat,
(C) 5-15 Gew.-% wasserunlösliche Phase,
(D) 5-20 Gew.-% Antiperspirant-/Desodorant-Wirkstoff,
(E) 20-40 Gew.-% Silikonöl, und
(F) 2-8 Gew.-% Silikonölemulgator, jeweils bezogen auf das Gesamtgewicht der Doppelemulsion,
wobei das Gewichtsverhältnis von (A) zu (B) mindestens 2.0 beträgt.

Bei einer O/W/O-Doppelemulsion liegt gegenüber einer W/O-Emulsion ohne innere Ölphase ein verringerter Wasseranteil vor, so dass sich die Emulsion nach dem Auftragen auf die Haut reichhaltiger anfühlt.

### Herstellung der erfindungsgemäßen Mikroemulsionen und Doppelemulsionen

Die Herstellung der Mikroemulsion erfolgt durch Vermischen der Inhaltsstoffe und Erwärmen bis alle Inhaltsstoffe geschmolzen sind. Bei dieser Temperatur wird üblicherweise für 30 Minuten mit moderater Rührenergie homogenisiert (Flügelrührer).

Die Herstellung der Doppelemulsion kann beispielsweise wie folgt erfolgen:
1. Vorlegen der Komponenten E und F
2. leichtes Erwärmen bei moderater Rührmechanik (Flügelrührer)
3. Zugabe der Mikroemulsion mit den Komponenten A,B,C,D
4. Homogenisieren bei moderater Rührmechanik (Flügelrührer)

### Formulierungen

Die Mikroemulsionen und Doppelemulsionen der vorliegenden Erfindung können in üblichen Darreichungsformen von Antitranspirant/Desodorant-Formulierungen vorliegen, bevorzugt in Form von Antitranspirant-/Deosprays, Antitranspirant-/Deolotionen, Antitranspirant-/Deoroli-ons, Antitranspirant-/Deogelen, Antitranspirant-/Deocremes oder Antitranspirant-/Deostiften.

### Beispiele

Die folgenden Beispiele sollen die vorliegende Erfindung näher erläutern.

Die Mikroemulsionen wurden wie oben beschrieben durch Vermischen der Inhaltsstoffe und Erwärmen bis alle Inhaltsstoffe geschmolzen sind unter Rühren für 30 Minuten mit moderater Rührenergie homogenisiert (Flügelrührer) hergestellt:
Die Doppelemulsionen wurden ebenfalls wie oben beschrieben hergestellt, d.h. durch Vorlage der Komponenten E und F, leichtem Erwärmen unter Rühren mit einem Flügelrührer, Zugabe der Mikroemulsion mit den Komponenten A,B,C,D und Homogenisieren mit einem Flügelrührer.
Die Mikroemulsionen und Doppelemulsionen wurden 4 Wochen bei Raumtemperatur (ca. 23°C) gelagert.
   Es erfolgte eine visuelle Beurteilung, ob eine Trübung oder Phasenseparation auftritt.
Tabellen 1 und 2 zeigen Zusammensetzungen und ermittelte Stabilitäten von erfindungsgemäßen Mikroemulsionen und von Vergleichszusammensetzungen. Insbesondere Tab. 1 zeigt klar, dass sämtliche Merkmale der Erfindung erfüllt sein müssen, um reproduzierbar eine Verbesserung der Stabilität der Mikroemulsionen zu erhalten.

**Tabelle 1: Zusammensetzungen und Stabilitäten von erfindungsgemäßen Mikroemulsionen und Vergleichszusammensetzungen**

| **SUBSTANZ** | **INCI** | **Bsp.1** | **Bsp.2** | **Vergl.-bsp. 1** | **Vergl.-bsp. 2** | **Vergl.-bsp. 3** | **Vergl.-bsp. 4** | **Vergl.-bsp. 5** | **Vergl.-bsp. 6** |
|---|---|---|---|---|---|---|---|---|---|
| Monomuls 90 O18 (B) | Glyceryl Oleate (100 %) | 7,52 | 8,00 | 3,00 | 6,00 | 6,00 | 8,00 | 0,02 | 0,04 |
| Plantacare 2000 UP (A) | Decyl Glucoside (53 %) | 32,90 | 36,00 | 26,42 | 15,09 | 52,83 | 38,30 | 0,28 | 0,28 |
| Cetiol OE (C) | Dicaprylyl Ether (100 %) | 18,80 | 20,00 | 15,00 | 15,00 | 15,00 | 15,00 | 0,5 | 30,00 |
| Locron L (Clariant) (D) | Aluminum Chlorohydrate (50 %) | 40,00 | 19,86 | 30,00 | 30,00 | 20,00 | 30,00 | 0,20 | 50,00 |
| Aqua dem | | 0,28 | 5,64 | 25,58 | 33,91 | 6,17 | 18,70 | 99,00 | 16,68 |
| | | | | | | | | | |
| **Stabilität** | | stabil | stabil | instabil | instabil | sehr zäh und klebrig | instabil | instabil | instabil |

### Tabelle 2 zeigt weitere erfindungsgemäße Beispiele für Mikroemulsionen und ihrer Stabilitäten.

**Tabelle 2: Zusammensetzungen und Stabilitäten weiterer erfindungsgemäßer Mikroemulsionen**

| **SUBSTANZ** | **INCI** | **Bsp.3** | **Bsp.4** |
|---|---|---|---|
| Monomuls 90 018 (B) | Glyceryl Oleate (100 %) | 6,4 | 5,6 |
| Plantacare 2000 UP (A) | Decyl Glucoside (53 %) | 28,00 | 24,50 |
| Cetiol OE (C) | Dicaprylyl Ether (100 %) | 16 | 14 |
| Rezal 36 GP Powder (Reheis) (D) | Aluminum Zirconium Tetrachlorohydrex-Gly (100 %) | 20,00 | 30,00 |
| | Benzoesäure (100%) | 0,40 | 0,35 |
| Aqua. dem | | 29,20 | 25,55 |
| | | | |
| pH-Wert | | 2,8-3,2 | 3,1 |
| **Stabilität** | | stabil | stabil |

Die Tabellen 3 und 4 zeigen weitere Formulierungsbeispiele und Charakterisierungen erfindungsgemäßer Mikroemulsionen (Tab. 3) und Doppelemulsionen (Tab. 4).

**Tab. 3 Zusammensetzungen und Eigenschaften weiterer erfindungsgemäßer Mikroemulsionen**

| **SUBSTANZ** | **INCI** | **Bsp. 5 (% AS)** | **Bsp. 6 (%AS)** |
|---|---|---|---|
| Monomuls 90 O18 (B) | Glyceryl Oleate | 6,4 | 6,4 |
| Plantacare 2000 UP (A) | Decyl Glucoside | 14,0 | 14,0 |
| Cetiol OE (C) | Dicaprylyl Ether | 16,0 | 16,0 |
| Locron L (Clariant) (D) | | - | 20,00 |
| Rezal 36 GP Powder (Reheis) (D) | Aluminum Zirconium Tetrachlorohydrex-Gly | 20,00 | - |
| | Citric Acid | 0,3 | 0,3 |
| Preservative | | q.s. | q.s. |
| Aqua. dem | | add 100 | add 100 |
| | | | |
| pH-Wert | | 3,7 | 2,8 |
| Aussehen | | transparent | transparent |

**Tab. 4 Zusammensetzungen und Eigenschaften erfindungsgemäßer Doppelemulsionen**

| **SUBSTANZ** | **INCI** | **Bsp. 7 (% AS)** | **Bsp.8 (% AS)** |
|---|---|---|---|
| Monomuls 90 O18 (B) | Glyceryl Oleate | 4,6 | 3,9 |
| Plantacare 2000 UP (A) | Decyl Glucoside | 10,0 | 8,6 |
| Cetiol OE (C) | Dicaprylyl Ether | 11,4 | 9,8 |
| Locron L (Clariant) (D) | | - | 10,00 |
| Rezal 36 GP Powder (Reheis) (D) | Aluminum Zirconium Tetrachlorohydrex-Gly | 10,00 | - |
| DC 245 (Dow Coming) (E) | Cyclopentasiloxane | 28,0 | 35,0 |
| Abil EM 90 (Evonik) (F) | Cetyl PEG/PPG-101 1-Dimethicone | 5,0 | 6,0 |
| | Citric Acid | 0,2 | 0,2 |
| Preservative | | q.s. | q.s. |
| Aqua. dem | | add 100 | add 100 |
| | | | |
| pH-Wert | | 4,0 | 3,5 |
| Aussehen | | transparent | leicht trüb |

Die Trübung im Fall von Bsp. 8 war nur sehr schwach, sodass auch diese Doppelemulsion als vorteilhaft im Sinne der Erfindung angesehen wurde.

## Patentansprüche

1. Antiperspirant/Desodorant-Mikroemulsion mit folgenden Bestandteilen:
(A) 10-20 Gew.-% Alkyl- und/oder Alkenyloligoglykosid(en),
(B) 5-10 Gew.-% Fettsäure-Polyol-Partialester,
(C) 10-30 Gew.-% Ölphase, und
(D) 5-30 Gew.-% Antiperspirant-/Desodorant-Wirkstoff, jeweils bezogen auf das Gesamtgewicht der Mikroemulsion,
wobei das Gewichtsverhältnis von (A) zu (B) mindestens 2.0 beträgt.

2. Mikroemulsion nach Anspruch 1, **dadurch gekennzeichnet, dass** das Gewichtsverhältnis von (A) zu (B) 2.0 bis 4.0 beträgt.

3. Mikroemulsion nach Anspruch 1 oder 2, **dadurch gekennzeichnet, dass** sie keine ethoxylierten Substanzen enthält.

4. Mikroemulsion nach einem der vorhergehenden Ansprüche, **dadurch gekennzeichnet, dass** die Ölphase (C) durch symmetrische oder unsymmetrische Dialkylether mit 6 bis 22 Kohlenstoffatomen pro Alkylgruppe, bevorzugt Dicaprylylether, gebildet ist.

5. Mikroemulsion nach einem der vorhergehenden Ansprüche, **dadurch gekennzeichnet, dass** das Alkyl- und/oder Alkenyl(oligo)glykosid 8 bis 22 Kohlenstoffatome im Alk(en)ylrest aufweist.

6. Mikroemulsion nach einem der vorhergehenden Ansprüche, **dadurch gekennzeichnet, dass** das Gewichtsverhältnis der Komponente (C) zur Komponente (B) mindestens 2.0 beträgt.

7. Mikroemulsion nach einem der vorhergehenden Ansprüche, **dadurch gekennzeichnet, dass** für Komponente (B) ein Glycerinoleat, bevorzugt ein Glycerinmonooleat , eingesetzt wird.

8. Doppelemulsion, welche die Mikroemulsion nach einem der Ansprüche 1 bis 7 als dispergierte Phase und eine Ölphase als äußere Phase enthält.

9. Doppelemulsion nach Anspruch 8, **dadurch gekennzeichnet, dass** die äußere Ölphase durch Silikonöl gebildet ist.

10. Doppelemulsion nach Anspruch 9, **dadurch gekennzeichnet, dass** sie einen Silikonölemulgator enthält.

11. Doppelemulsion nach einem der Ansprüche 8 bis 10, **dadurch gekennzeichnet, dass** sie
(A) 6-15 Gew.-% Alkyl- und/oder Alkenyloligoglykosid(e)n (APG),
(B) 3-6 Gew.-% Fettsäure-Polyol-Partialester,
(C) 5-15 Gew.-% wasserunlösliche Phase,
(D) 5-20 Gew.-% Antiperspirant-/Desodorant-Wirkstoff,
(E) 20-40 Gew.-% Silikonöl, und
(F) 2-8 Gew.-% Silikonölemulgator, jeweils bezogen auf das Gesamtgewicht der Doppelemulsion, enthält,
wobei das Gewichtsverhältnis von (A) zu (B) mindestens 2.0 beträgt.

12. Mikroemulsionen oder Doppelemulsionen nach einem der vorhergehenden Ansprüche in Form von Antitranspirant-/Deosprays, Anlitranspirant-/Deolotionen, Antitranspirant-/Deoroll-ons, Antitranspirant-/Deogelen, Antitranspirant-/Deocremes oder Antitranspirant/Deostiften.

13. Verfahren zur Verbesserung der Stabilität von Mikroemulsionen, welche keine ethoxylierten Verbindungen erhalten, **dadurch gekennzeichnet, dass** man die Komponenten wie folgt einstellt:
(A) 10-20 Gew.-% Alkyl- und/oder Alkenyloligoglykosid(e),
(B) 5-10 Gew.-% Fettsäure-Polyol-Partialester,
(C) 10-30 Gew.-% Ölphase, und
(D) 5-30 Gew.-% Antiperspirant-/Desodorant-Wirkstoff, jeweils bezogen auf das Gesamtgewicht der Mikroemulsion,
wobei das Gewichtsverhältnis von (A) zu (B) mindestens 2.0 beträgt.

14. Verfahren nach Anspruch 13, **dadurch gekennzeichnet, dass** man das Gewichtsverhältnis von (A) zu (B) auf 2.0 bis 4.0 einstellt.

15. Verfahren nach Anspruch 13 oder 14, **dadurch gekennzeichnet, dass** man das Gewichtsverhältnis von (C) zu (B) auf 2.0 oder größer, bevorzugt 2.0 bis 2.5 einstellt.
